# EUROPEAN PATENT APPLICATION

(11) **EP 3 760 646 A1**
(43) Date of publication of application: **06.01.2021**
(21) Application number: 19760968.8
(22) Date of filing: 28.02.2019
(51) Int. Cl.: C08B 30/18, C07C 35/44, C07C 39/16, C07C 211/38, C08G 18/32, C08G 81/00

(54) **POLYMER MATERIAL AND METHOD FOR MANUFACTURING SAME**

(30) Priority: 01.03.2018 JP 2018036986
(71) Applicant: Osaka University, Suita-shi, Osaka 565-0871 (JP)
(72) Inventor: HARADA, Akira, Suita-shi, Osaka 565-0871 (JP); TAKASHIMA, Yoshinori, Suita-shi, Osaka 565-0871 (JP); ZHENG, Yongtai, Suita-shi, Osaka 565-0871 (JP); KOBAYASHI, Yuichiro, Suita-shi, Osaka 565-0871 (JP)
(74) Representative: Vossius & Partner Patentanwälte Rechtsanwälte mbB
(86) International application number: PCT/JP2019/007945
(87) International publication number: WO 2019/168128

(57) **Abstract**

Provided are a macromolecular material that can form various structures, and that exhibits excellent toughness and strength; and a method for producing the macromolecular material. The macromolecular material includes in a polymer chain thereof a structural unit represented by the following formula (1):

-R¹-R^{H} (1)

(R¹ represents NH or O, and R^{H} represents a host group), wherein the host group is a monovalent group formed by removing one hydrogen atom or hydroxy group from a cyclodextrin derivative, and the cyclodextrin derivative has a structure formed such that a hydrogen atom of at least one hydroxy group contained in a cyclodextrin is replaced with at least one group selected from the group consisting of a hydrocarbon group, an acyl group, and -CONHR (R represents a methyl group or an ethyl group).

## Description

### Technical Field

The present invention relates to a macromolecular material, and a method for producing the macromolecular material.

### Background Art

Supramolecular materials that are highly functionalized in various aspects have been actively developed by sophisticatedly using non-covalent-bond interaction, such as host-guest interaction. For example, PTL 1 discloses a macromolecular material that uses host-guest interaction. Such a macromolecular material that uses host-guest interaction has received attention from various fields as a novel material due to its unprecedented, excellent properties, such as excellent mechanical properties.

### Citation List

### Patent Literature

PTL 1: WO2015/030079

### Summary of Invention

### Technical Problem

There has been demand for macromolecular materials to which various functionalities are added. For example, the development of macromolecular materials that exhibit further improved mechanical properties is strongly demanded. In this regard, for example, conventional macromolecular materials that use host-guest interaction have limitations in terms of altering the composition of the materials; e.g., resin materials such as urethanes are difficult to modify by using host-guest interaction. This is because conventional macromolecular materials that use host-guest interaction contain a polymer formed of a monomer with a relatively high water-solubility as the main component, and are thus restricted by the low degree of freedom in structural design and material design.

The present invention was made in view of these circumstances in the art. An object of the invention is to provide a macromolecular material that can form various structures, and that exhibits excellent toughness and strength; and a method for producing the macromolecular material.

### Solution to Problem

The present inventors conducted extensive research to achieve the object, and found that introducing a host-group-containing structural unit of a specific structure can achieve the object. The present inventors then completed the invention.

Specifically, the present invention includes, for example, the subject matter described in the following items.
Item 1
   A macromolecular material comprising in a polymer chain thereof a structural unit represented by the following formula (1) :

   -R¹-R^{H} (1)

   wherein R¹ represents NH or O, and R^{H} represents a host group,
   wherein
   the host group is a monovalent group formed by removing one hydrogen atom or hydroxy group from a cyclodextrin derivative, and
   the cyclodextrin derivative has a structure formed such that a hydrogen atom of at least one hydroxy group contained in a cyclodextrin is replaced with at least one group selected from the group consisting of a hydrocarbon group, an acyl group, and -CONHR wherein R represents a methyl group or an ethyl group.
Item 2
   The macromolecular material according to Item 1, wherein the acyl group is an acetyl group.
Item 3
   The macromolecular material according to Item 1 or 2, wherein the host group includes a guest group to form a clathrate complex, and the guest group has a structure such that the guest group is chemically bonded to the polymer chain.
Item 4
   The macromolecular material according to Item 1 or 2, having a structure such that the polymer chain penetrates through a ring of the host group.
Item 5
   A method for producing a macromolecular material comprising reacting a clathrate compound with a polyfunctional compound,
   the clathrate compound containing
   a compound represented by the following formula (2):

      R²-R^{H} (2)

      wherein R² represents NH₂ or OH, and R^{H} represents a host group and
   a guest molecule,
   wherein
   the host group is a monovalent group formed by removing one hydrogen atom or hydroxy group from a cyclodextrin derivative, and
   the cyclodextrin derivative has a structure formed such that a hydrogen atom of at least one hydroxy group contained in a cyclodextrin is replaced with at least one group selected from the group consisting of a hydrocarbon group, an acyl group, and -CONHR wherein R represents a methyl group or an ethyl group.
Item 5-1
   A method for producing the macromolecular material of any one of Items 1 to 4, comprising reacting a clathrate compound with a polyfunctional compound,
   the clathrate compound containing
   a compound represented by the following formula (2):

      R²-R^{H} (2)

      wherein R² represents NH₂ or OH, and R^{H} represents a host group, and
   a guest molecule,
   wherein
   the host group is a monovalent group formed by removing one hydrogen atom or hydroxy group from a cyclodextrin derivative, and
   the cyclodextrin derivative has a structure formed such that a hydrogen atom of at least one hydroxy group contained in a cyclodextrin is replaced with at least one group selected from the group consisting of a hydrocarbon group, an acyl group, and -CONHR wherein R represents a methyl group or an ethyl group.
Item 6
   The method for producing a macromolecular material according to Item 5, wherein the acyl group is an acetyl group.
Item 7
   The method for producing a macromolecular material according to Item 5 or 6, wherein the guest molecule is at least one member selected from the group consisting of compounds having one or two amino groups, and compounds having one or two hydroxy groups.

### Advantageous Effects of Invention

The macromolecular material according to the present invention forms various structures, and exhibits excellent toughness and strength.

The method for producing a macromolecular material according to the present invention produces a macromolecular material that forms various structures, and that exhibits excellent toughness and strength in a simple manner.

### Brief Description of Drawings

Fig. 1 is a schematic diagram that illustrates examples of the macromolecular material according to the present invention.
Fig. 2 is a schematic diagram that illustrates another example of the macromolecular material according to the present invention.
Fig. 3(a) and Fig. 3(b) are respectively a mass spectrum and an NMR spectrum of the OHβCDOMe obtained in Production Example 1.
Fig. 4 (a) and Fig. 4 (b) are respectively a mass spectrum and an NMR spectrum of NH₂βCDOMe obtained in Production Example 2.
Fig. 5 illustrates the results of a tensile test performed on the macromolecular materials obtained in Example 1 and Comparative Example 1.
Fig. 6 illustrates a self-healing properties test performed on the macromolecular material obtained in Example 1 and the outcome of the test.
Fig. 7 illustrates the results of a tensile test performed on the macromolecular materials obtained in Example 2 and Comparative Example 2.
Fig. 8 illustrates a self-healing properties test performed on the macromolecular material obtained in Example 2 and the outcome of the test.
Fig. 9 illustrates the results of a tensile test performed on the macromolecular materials obtained in Example 3 and Comparative Example 3.
Fig. 10 illustrates the results of a tensile test performed on the macromolecular materials obtained in Example 4 and Comparative Example 4.
Fig. 11(a) illustrates a tensile-load curve of a test specimen of the macromolecular material obtained in Example 4 after healing, and Fig. 11(b) illustrates the results of the application of rupture stress before cutting and after healing the macromolecular material obtained in Example 4.
Fig. 12 illustrates the results of a tensile test performed on the macromolecular materials obtained in Example 5 and Comparative Example 5.
Fig. 13 illustrates a self-healing properties test performed on the macromolecular material obtained in Example 5 and the results of the test.
Fig. 14(a) illustrates a tensile-load curve of a test specimen of the macromolecular material obtained in Example 5 after healing, and Fig. 14(b) illustrates the results of the application of rupture stress before cutting and after healing the macromolecular material obtained in Example 5.

### Description of Embodiments

Below, embodiments of the present invention are described in detail. The terms "comprise," "contain," and "include" in the present specification include the concepts of "comprise," "contain," "include," "consist essentially of," and "consist of."

### 1. Macromolecular Material

The macromolecular material according to the present invention contains a structural unit represented by the following formula (1) in its polymer chain.

-R¹-R^{H} (1)

In formula (1), R¹ represents NH or O.

In formula (1), R^{H} represents a host group.

The host group is a monovalent group formed by removing one hydrogen atom or hydroxy group from a cyclodextrin derivative. The cyclodextrin derivative has a structure formed such that the hydrogen atom of at least one hydroxy group contained in a cyclodextrin is replaced with at least one group selected from the group consisting of a hydrocarbon group, an acyl group, and -CONHR wherein R represents a methyl group or an ethyl group.

The macromolecular material, in particular due to the structural unit represented by formula (1), can form various structures, and exhibit excellent toughness and strength. The structural unit represented by formula (1) contains a host group essential to host-guest interaction, and can form host-guest interaction reversible intramolecularly or intermolecularly when a guest group is introduced into the polymer chain. This can increase, for example, the toughness and strength of the macromolecular material. Additionally, a macromolecular material formed to have a specific structure as described later can self-heal, for example, even if the macromolecular material is cut; this is because adhering the cross-sections of the macromolecular material to each other allows the cross-sections to form host-guest interaction again. More specifically, a macromolecular material formed to have a specific structure exhibits self-healing properties when being cut.

The cyclodextrin derivative is at least one member selected from the group consisting of α-cyclodextrin derivatives, β-cyclodextrin derivatives, and γ-cyclodextrin derivatives. The cyclodextrin derivative as used in the present specification refers to a molecule with a structure formed such that a cyclodextrin molecule is substituted with a different organic group. Just to note, "cyclodextrin" in the present specification refers to at least one member selected from the group consisting of α-cyclodextrin, β-cyclodextrin, and γ-cyclodextrin.

The host group is, as described above, a monovalent group formed by removing one hydrogen atom or hydroxy group from a cyclodextrin derivative. The removed hydrogen atom or hydroxy group may be of any site of the cyclodextrin derivative. From the standpoint of ease of forming a host group, the host group is preferably a monovalent group formed by removing one hydroxy group from a cyclodextrin derivative.

The cyclodextrin derivative for forming a host group, as described above, has a structure formed such that at least one hydroxy group contained in a cyclodextrin is substituted with at least one group selected from the group consisting of a hydrocarbon group, an acyl group, and -CONHR wherein R represents a methyl group or an ethyl group. Due to this structure, the structural unit represented by formula (1) and a monomer for forming the structural unit can have, for example, high affinity for both a hydrophilic monomer unit and a hydrophobic monomer unit. This enables the macromolecular material to form copolymers of various monomer units. In the present specification, "at least one group selected from the group consisting of a hydrocarbon group, an acyl group, and -CONHR wherein R represents a methyl group or an ethyl group" may be referred to as "a hydrocarbon group etc." for convenience.

When the total number of hydroxy groups in a single molecule of a cyclodextrin is N, N of α-cyclodextrin is 18, N of β-cyclodextrin is 21, and N of γ-cyclodextrin is 24.

If the host group is a monovalent group formed by removing one "hydroxy group" from a cyclodextrin derivative, the maximum number of hydroxy groups whose hydrogens can be replaced with a hydrocarbon group etc. is N-1 per molecule of the cyclodextrin derivative. If the host group is a monovalent group formed by removing one "hydrogen atom" from a cyclodextrin derivative, the maximum number of hydroxy groups whose hydrogens can be replaced with a hydrocarbon group etc., is N per molecule of the cyclodextrin derivative.

The host group preferably has a structure such that the hydrogen atoms of at least 70% of the total number of hydroxy groups per molecule of the cyclodextrin derivative are replaced with a hydrocarbon group etc. In this case, the structural unit represented by formula (1) can exhibit higher affinity for hydrophobic monomer units. The host group more preferably has a structure such that the hydrogen atoms of at least 80% of the total number of hydroxy groups per molecule of the cyclodextrin derivative are replaced with a hydrocarbon group etc.; and particularly preferably has a structure such that the hydrogen atoms of at least 90% of the total number of hydroxy groups per molecule of the cyclodextrin derivative are replaced with a hydrocarbon group etc.

The host group preferably has a structure such that the hydrogen atoms of at least 13 hydroxy groups out of the total number of hydroxy groups per molecule of α-cyclodextrin derivative are replaced with a hydrocarbon group etc. In this case, the structural unit represented by formula (1) can exhibit higher affinity for hydrophobic monomer units. The host group more preferably has a structure such that the hydrogen atoms of at least 15 hydroxy groups out of the total number of hydroxy groups per molecule of α-cyclodextrin derivative are replaced with a hydrocarbon group etc.; and particularly preferably has a structure such that the hydrogen atoms of at least 17 hydroxy groups out of the total number of hydroxy groups per molecule of α-cyclodextrin derivative are replaced with a hydrocarbon group etc.

The host group preferably has a structure such that the hydrogen atoms of at least 15 hydroxy groups out of the total number of hydroxy groups per molecule of β-cyclodextrin derivative are replaced with a hydrocarbon group etc. In this case, the structural unit represented by formula (1) can exhibit higher affinity for hydrophobic monomer units. The host group more preferably has a structure such that the hydrogen atoms of at least 17 hydroxy groups out of the total number of hydroxy groups per molecule of β-cyclodextrin derivative are replaced with a hydrocarbon group etc.; and particularly preferably has a structure such that the hydrogen atoms of at least 19 hydroxy groups out of the total number of hydroxy groups per molecule of β-cyclodextrin derivative are replaced with a hydrocarbon group etc.

The host group preferably has a structure such that the hydrogen atoms of at least 17 hydroxy groups of the total number out of hydroxy groups per molecule of γ-cyclodextrin derivative are replaced with a hydrocarbon group etc. In this case, the structural unit represented by formula (1) can exhibit higher affinity for hydrophobic monomer units. The host group more preferably has a structure such that the hydrogen atoms of at least 19 hydroxy groups out of the total number of hydroxy groups per molecule of γ-cyclodextrin derivative are replaced with a hydrocarbon group etc.; and particularly preferably has a structure such that the hydrogen atoms of at least 21 hydroxy groups out of the total number of hydroxy groups per molecule of γ-cyclodextrin derivative are replaced with a hydrocarbon group etc.

The type of the hydrocarbon group is not particularly limited. Examples of the hydrocarbon group include an alkyl group, an alkenyl group, and an alkynyl group.

The number of carbon atoms of the hydrocarbon group is not particularly limited. The number of carbon atoms of the hydrocarbon group is preferably 1 to 4 because such a structural unit represented by formula (1) exhibits higher affinity for both hydrophilic monomer units and hydrophobic monomer units, and easily forms host-guest interaction.

Specific examples of hydrocarbon groups having 1 to 4 carbon atoms include a methyl group, an ethyl group, a propyl group, and a butyl group. When the hydrocarbon group is a propyl group or a butyl group, the hydrocarbon group may be linear or branched.

The hydrocarbon group may be substituted, as long as the effects of the present invention are not impaired.

Examples of the acyl group include an acetyl group, a propionyl group, and a formyl group. The acyl group may be further substituted. The acyl group is preferably an acetyl group; this is because such a structural unit represented by formula (1) exhibits higher affinity for both hydrophilic monomer units and hydrophobic monomer units, and easily forms host-guest interaction; additionally, a macromolecular material excellent in toughness and strength can be easily obtained.

-CONHR wherein R represents a methyl group or an ethyl group is a methyl carbamate group or an ethyl carbamate group. -CONHR is preferably an ethyl carbamate group because such a structural unit represented by formula (1) exhibits higher affinity for both hydrophilic monomer units and hydrophobic monomer units, and easily forms host-guest interaction.

The macromolecular material contains a structural unit other than the structural unit represented by formula (1). Below, the structural unit other than the structural unit represented by formula (1) is simply referred to as "second structural unit."

The second structural unit can be any structural unit. Examples of the second structural unit include structural units having a urethane bond.

The structural units having a urethane bond can be any structural unit, and can be selected from a wide range of structural units present in known urethane resins. An example of a structural unit having a urethane bond is a structural unit formed such that a compound unit having two isocyanate groups bonds to a compound unit having two hydroxy groups.

Examples of compounds having two isocyanate groups include a compound represented by the following formula (3):

O=C=N-R^{A}-N=C=O (3)

wherein R^{A} represents a divalent organic group.

Specific examples of R^{A} (divalent organic group) in formula (3) include (CH₂)ₙ (n is an integer of 1 to 20), methyl diphenylene, methyl dicyclohexylene, 3-methyl-3,5,5-trimethyl cyclohexylene, dimethyl phenylene, tolylene, phenylene, benzylidene, cyclohexylene, and isophorone. When the divalent organic group is (CH₂)ₙ, n is preferably 2 to 10, and more preferably 3 to 8.

Examples of compounds having two hydroxy groups include a compound represented by the following formula (4):

HO-(CH₂CH₂O)ₘ-OH (4)

wherein m represents an integer of 1 to 50.

In formula (3), m is preferably 1 to 30, more preferably 2 to 20, and particularly preferably 2 to 10.

When the compound having two isocyanate groups is a compound represented by formula (3), and the compound having two hydroxy groups is a compound represented by formula (4), the structural unit having a urethane bond formed by the bond between these compounds is represented by the following formula (34).

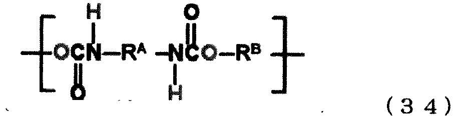

In formula (34), R^{A} is synonymous with R^{A} in formula (3), and R^{B} is synonymous with (CH₂CH₂O)ₘ in formula (4).

The macromolecular material may contain "a third structural unit" other than the structural unit represented by formula (1) and the second structural unit. An example of the third structural unit is a structural unit capable of crosslinking polymer chains (i.e., a three-dimensional network structure).

Examples of the third structural unit include structural units formed by removing all of the hydrogen atoms of three or more hydroxy groups in a compound having three or more hydroxy groups, and structural units derived from a compound having three or more isocyanate groups.

Examples of types of compounds having three or more hydroxy groups include triethanolamine, trimethylolpropane, and 1,3,5-tris(2-hydroxyethyl)isocyanurate. Additionally, compounds having three or more hydroxy groups for use may be selected from a wide range of known compounds having three or more hydroxy groups, such as PEG-core dendrimer (Sigma-Aldrich), Multi-arm PEGs (Sigma-Aldrich), Y-shape PEG (Sigma-Aldrich), high-purity multi-arm PEG (NOF Corporation), branched PEG derivatives (NOF Corporation), and SUNBRIGHT (registered trademark) series polyethylene glycol modifier (Yuka Sangyo Co., Ltd.).

Examples of types of compounds having three or more isocyanate groups include tris-(tolueneisocyanate)isocyanurate, and tris-(hexamethyleneisocyanate)isocyanurate. Additionally, known biuret-type, isocyanurate-type, or adduct-type isocyanate compounds are also widely usable.

The compound having three or more hydroxy groups preferably has 3 to 8 hydroxy groups, more preferably 3 or 4 hydroxy groups, and particularly preferably 3 hydroxy groups.

The macromolecular material that contains the third structural unit can form a crosslinked structure.

The macromolecular material may contain, in addition to the structural unit represented by formula (1), either the second structural unit or the third structural unit, or both. The macromolecular material may consist of the structural unit represented by formula (1) and the second structural unit. The macromolecular material may consist of the structural unit represented by formula (1) and the third structural unit. The macromolecular material may consist of the structural unit represented by formula (1), the second structural unit, and the third structural unit.

In the macromolecular material, the host group contained in the structural unit represented by formula (1) includes a guest group to form a clathrate complex, and the guest group can chemically bond to the polymer chain. More specifically, the guest group can chemically bond to the main chain or a side chain of the polymer chain.

Examples of guest groups include guest molecules capable of forming a clathrate complex with the host group. To be precise, the guest group is a group formed by removing one or two hydrogen atoms from a guest molecule.

The guest molecule can be any molecule that can form a clathrate complex with the host group. Examples of guest groups include compounds having one or two amino groups, compounds having one or two hydroxy groups, compounds having one or two carboxy groups, compounds having one or two epoxy groups, compounds having one or two isocyanate groups, compounds having one or two thiol groups, and compounds having one or two carboxylic acid chloride.

Examples of compounds having one or two amino groups include 1-adamantaneamine, benzyl amine, tert-butylamine, n-butylamine, 1-aminopyrene, aminoferrocene, 4-aminoazobenzene, 4-aminostilbene, cyclohexylamine, hexylamine, 4,4'-diaminodiphenylmethane, p-xylylenediamine, diaminoferrocene, 4,4'-diaminoazobenzene, 4,4'-diaminostilbene, 1,4-diaminocyclohexane, 1,6-diaminocyclohexane, α,ω-diaminopolyethylene glycol, α,ω-diaminopolypropylene glycol, 2,2-bis(4-aminophenyl)propanel,1-bis(4-aminophenyl)-1-phenylethane, 2,2-bis(4-aminophenyl)hexafluoropropane, 2,2-bis(4-aminophenyl)butane, bis(4-aminophenyl)diphenylmethane, 2,2-bis(3-methyl-4-aminophenyl)propane, bis(4-aminophenyl)-2,2-dichloroethylene, 1,1-bis(4-aminophenyl)ethane, 2,2-bis(4-amino-3-isopropyl phenyl)propane, 1,3-bis(2-(4-aminophenyl)-2-propyl)benzene, bis(4-aminophenyl)sulfone, 1,4-bis(2-(4-aminophenyl)-2-propyl)benzene, 5,5'-(1-methylethylidene)-bis[1,1'-(bisphenyl)-2-amine]propane, 1,1-bis(4-aminophenyl)-3,3,5-trimethylcyclohexane, and 1,1-bis(4-aminophenyl)cyclohexane.

Examples of compounds having one or two hydroxy groups include 1-hydroxyadamantane, benzyl alcohol, tert-butyl alcohol, n-butyl alcohol, 1-hydroxypyrene, 1-hydroxymethyl ferrocene, 4-hydroxy azobenzene, 4-hydroxy stilbene, cyclohexanol, hexanol, 4,4'-dihydroxydiphenylmethane, 2,2-bis(4-hydroxyphenyl)propane, 1,4-benzenedimethanol, 1,1'-dihydroxymethyl ferrocene, 4,4'-dihydroxy azobenzene, 4,4'-dihydroxy stilbene, 1,4-cyclohexanol, 1,6-hexanediol, polyethylene glycol, polypropylene glycol, 1,1-bis(4-hydroxyphenyl)-1-phenylethane, 2,2-bis(4-hydroxyphenyl)hexafluoropropane, 2,2-bis(4-hydroxyphenyl)butane, bis(4-hydroxyphenyl)diphenyl methane, 2,2-bis(3-methyl-4-hydroxyphenyl)propane, bis(4-hydroxyphenyl)-2,2-dichloroethylene, 1,1-bis(4-hydroxyphenyl)ethane, 2,2-bis(4-hydroxy-3-isopropyl phenyl)propane, 1,3-bis(2-(4-hydroxyphenyl)-2-propyl)benzene, bis(4-hydroxyphenyl)sulfone, 1,4-bis(2-(4-hydroxyphenyl)-2-propyl)benzene, 5,5'-(1-methylethylidene)-bis[1,1'-(bisphenyl)-2-hydroxy]propane, 1,1-bis(4-hydroxyphenyl)-3,3,5-trimethylcyclohexane, 1,1-bis(4-hydroxyphenyl)cyclohexane, and bisphenol A.

Examples of compounds having one or two carboxy groups include 1-carboxy adamantane, benzoic acid, pivalic acid, butanoic acid, 1-carboxypyrene, 1-carboxy ferrocene, 4-carboxy azobenzene, 4-carboxy stilbene, cyclohexanoic acid, hexanoic acid, 4,4'-dicarboxy diphenyl methane, 1,4-benzene dicarboxylic acid, 1,4-phenylenediacetic acid, 1,1'-dicarboxy ferrocene, 4,4'-dicarboxy azobenzene, 4,4'-dicarboxy stilbene, 1,4-cyclohexane dicarboxylic acid, 1,6-hexane dicarboxylic acid, α,ω-dicarboxy polyethylene glycol, α,ω-dicarboxy polypropylene glycol, 2,2-bis(4-carboxyphenyl)propane, 1,1-bis(4-carboxyphenyl)-1-phenylethane, 2,2-bis(4-carboxyphenyl)hexafluoropropane, 2,2-bis(4-carboxyphenyl)butane, bis(4-carboxyphenyl)diphenyl methane, 2,2-bis(3-methyl-4-carboxyphenyl)propane, bis(4-carboxyphenyl)-2,2-dichloroethylene, 1,1-bis(4-carboxyphenyl)ethane, 2,2-bis(4-carboxy 3-isopropyl phenyl)propane, 1,3-bis(2-(4-carboxyphenyl)-2-propyl)benzene, bis(4-carboxyphenyl)sulfone, 1,4-bis(2-(4-carboxyphenyl)-2-propyl)benzene, 5,5'-(1-methylethylidene)-bis[1,1'-(bisphenyl)-2-carboxy]propane, 1,1-bis(4-carboxyphenyl)-3,3,5-trimethylcyclohexane, and 1,1-bis(4-carboxyphenyl)cyclohexane.

Examples of compounds having one or two carboxylic acid chloride include 1-adamantane carbonyl chloride, terephthaloyl chloride, trimethyl acetyl chloride, butyryl chloride, 1-pyrene carbonyl chloride, 1-ferrocene carbonyl chloride, 4-azobenzene carbonyl chloride, 4-stilbene carbonyl chloride, cyclohexane carbonyl chloride, hexyl chloride, 4,4'-diphenylmethane dicarbonyl chloride, 1,4-benzenedicarbonyl chloride, 1,4-phenylene dicarbonyl chloride, 1,1'-ferrocene dicarbonyl chloride, 4,4'-azobenzene dicarbonyl chloride, 4,4'-stilbene carbonyl chloride, 1,4-cyclohexane dicarbonyl chloride, 1,6-hexane dicarbonyl chloride, α,ω-polyethylene glycol dicarbonyl chloride, α,ω-polypropylene glycol dicarbonyl chloride, 2,2-bis(4-phenylcarbonylchloride)propane, 1,1-bis(4-phenylcarbonylchloride)-1-phenylethane, 2,2-bis(4-phenylcarbonylchloride)hexafluoropropane, 2,2-bis(4-phenylcarbonylchloride)butane, bis(4-phenylcarbonylchloride)diphenyl methane, 2,2-bis(3-methyl-4-phenylcarbonylchloride)propane, bis(4-phenylcarbonylchloride)-2,2-dichloroethylene, 1,1-bis(4-phenylcarbonylchloride)ethane, 2,2-bis(3-isopropylphenyl-4-carbonylchloride)propane, 1,3-bis(2-(4-phenylcarbonylchloride)-2-propyl)benzene, bis(4-phenylcarbonylchloride)sulfone, 1,4-bis(2-(4-phenylcarbonylchloride)-2-propyl)benzene, 5,5'-(1-methylethylidene)-bis[1,1'-(bisphenyl)-2-carbonyl chloride]propane, 1,1-bis(4-phenylcarbonylchloride)-3,3,5-trimethylcyclohexane, and 1,1-bis(4-phenylcarbonylchloride)cyclohexane.

Examples of compounds having one or two epoxy groups include adamantane oxide, styrene oxide, 1,2-epoxy butane, 1-epoxy pyrene, epoxy ferrocene, 4-epoxy azobenzene, 4-epoxy stilbene, cyclohexyl oxide, 1,2-epoxy hexane, 2,2'-bis(4-glycidyloxyphenyl)propane, p-diglycidyloxybenzene, diglycidyloxyferrocene, 4,4'-diglycidyloxyazobenzene, 4,4'-diglycidyloxyferrocene, 1,4-diglycidyloxycyclohexane, 1,6-diglycidyloxycyclohexane, α,ω-diglycidyloxypolyethylene glycol, α,ω-diglycidyloxypolypropylene glycol, 1,1-bis(4-glycidyloxyphenyl)-1-phenylethane, 2,2-bis(4-glycidyloxyphenyl)hexafluoropropane, 2,2-bis(4-glycidyloxyphenyl)butane, bis(4-glycidyloxyphenyl)diphenyl methane, 2,2-bis(3-methyl-4-glycidyloxyphenyl)propane, bis(4-glycidyloxyphenyl)-2,2-dichloroethylene, 1,1-bis(4-glycidyloxyphenyl)ethane, 2,2-bis(4-glycidyloxy-3-isopropyl phenyl)propane, 1,3-bis(2-(4-glycidyloxyphenyl)-2-propyl)benzene, bis(4-glycidyloxyphenyl)sulfone, 1,4-bis(2-(4-glycidyloxyphenyl)-2-propyl)benzene, 5,5'-(1-methylethylidene)-bis[1,1'-(bisphenyl)-2-glycidyloxy]propane, 1,1-bis(4-glycidyloxyphenyl)-3,3,5-trimethylcyclohexane, and 1,1-bis(4-glycidyloxyphenyl)cyclohexane.

Examples of compounds having one or two isocyanate groups include 1-adamantane isocyanate, benzyl isocyanate, phenyl isocyanate, tert-butyl isocyanate, butyl isocyanate, 1-pyrene isocyanate, ferrocene isocyanate, azobenzene-4-isocyanate, stilbene-4-isocyanate, cyclohexane isocyanate, hexane isocyanate, 4,4'-diisocyanate phenyl methane, p-benzene diisocyanate, ferrocene-1,1'-diisocyanate, azobenzene-4,4'-diisocyanate, stilbene-4,4'-diisocyanate, cyclohexane-1,4-diisocyanate, cyclohexane-1,6-diisocyanate, polyethylene glycol diisocyanate, polypropylene glycol diisocyanate, 2,2-bis(4-phenylisocyanate)propane, 1,1-bis(4-phenylisocyanate)-1-phenylethane, 2,2-bis(4-phenylisocyanate)hexafluoropropane, 2,2-bis(4-phenylisocyanate)butane, bis(4-phenylisocyanate)diphenyl methane, 2,2-bis(3-methyl-4-phenylisocyanate)propane, bis(4-phenylisocyanate)-2,2-dichloroethylene, 1,1-bis(4-phenylisocyanate)ethane, 2,2-bis(3-isopropyl-4-phenylisocyanate)propane, 1,3-bis(2-(4-phenylisocyanate-)-2-propyl)benzene, bis(4-phenylisocyanate-)sulfone, 1,4-bis(2-(4-phenylisocyanate-)-2-propyl)benzene, 5,5'-(1-methylethylidene)-bis[1,1'-(bisphenyl)-2-isocyanate]propane, 1,1-bis(4-phenylisocyanate-)-3,3,5-trimethylcyclohexane, and 1,1-bis(4-phenylisocyanate-)cyclohexane.

Examples of compounds having one or two thiol groups include 1-adamantane thiol, benzyl thiol, tert-mercaptan, butane thiol, 1-thiol pyrene, ferrocene thiol, 4-thioazobenzene, 4-thiostilbene, cyclohexyl thiol, hexane thiol, 4,4'-dithiophenyl methane, p-benzene dithiol, 1,1'-dithioferrocene, 4,4'-dithioazobenzene, 4,4'-dithiostilbene, 1,4-dithiocyclohexane, 1,6-dithiocyclohexane, α,ω-dithiopolyethylene glycol, α,ω-dithiopolypropylene glycol, 1,1-bis(4-thiophenyl)-1-phenylethane, 2,2-bis(4-thiophenyl)hexafluoropropane, 2,2-bis(4-thiophenyl)butane, bis(4-thiophenyl)diphenyl methane, 2,2-bis(3-methyl-4-thiophenyl)propane, bis(4-thiophenyl)-2,2-dichloroethylene, 1,1-bis(4-thiophenyl)ethane, 2,2-bis(4-thio-3-isopropylphenyl)propane, 1,3-bis(2-(4-thiophenyl)-2-propyl)benzene, bis(4-thiophenyl)sulfone, 1,4-bis(2-(4-thiophenyl)-2-propyl)benzene, 5,5'-(1-methylethylidene)-bis[1,1'-(bisphenyl)-2-thiol]propane, 1,1-bis(4-thiophenyl)-3,3,5-trimethylcyclohexane, and 1,1-bis(4-thiophenyl)cyclohexane.

The guest molecule is preferably at least one member selected from the group consisting of 1-hydroxyadamantane (adamantanol), 1-adamantaneamine, 4,4'-dihydroxydiphenylmethane, 4,4'-diaminodiphenylmethane, polyethylene glycol, polypropylene glycol, α,ω-diaminopolyethylene glycol, and α,ω-diaminopolypropylene glycol.

The guest molecule is preferably at least one member selected from the group consisting of compounds having one or two amino groups, and compounds having one or two hydroxy groups. In this case, it is easy to produce a macromolecular material, and the macromolecular material is likely to exhibit improved self-healing properties or mechanical properties.

In the macromolecular material, the host group includes a guest group to form a clathrate complex, and the guest group can chemically bond to the polymer chain of the macromolecular material. The macromolecular material is classified into the following two modes according to the way the guest group bonds to the polymer chain. Below, these modes are referred to as a macromolecular material of the first embodiment, and a macromolecular material of the second embodiment.

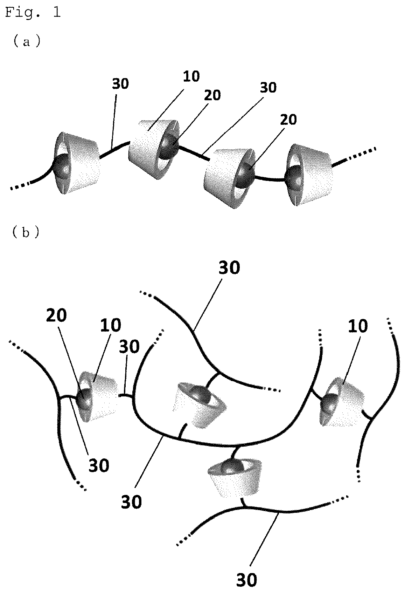

Fig. 1(a) is a schematic diagram that illustrates a macromolecular material of the first embodiment. In the macromolecular material of the first embodiment, guest groups 20 are bound to polymer chain 30. More specifically, in the macromolecular material of the first embodiment, guest groups 20 are included in host groups 10, thereby forming clathrate complexes, and one end of each guest group 20 is bound to polymer chain 30 that forms the macromolecular material. Additionally, although no shown in Fig. 1, R¹ bound to host group 10 is bound to polymer chain 30. In other words, the structural unit represented by formula (1) R^{H}-R¹- is bound to polymer chain 30 in the macromolecular material of the first embodiment.

In the macromolecular material of the first embodiment, repeating the bond of "polymer chain 30"-"guest group 20"---"R^{H}-R¹"-"polymer chain 30" forms a polymer chain, and thus forms the macromolecular material. As used here, "guest group 20"---"R^{H}-R¹" means that guest group 20 forms a clathrate complex with R^{H}. The macromolecular material of the first embodiment has a structure in which the terminals of polymer chains 30 are crosslinked; thus, the macromolecular material can be particularly referred to as "terminal-crosslinked macromolecular material."

In the macromolecular material of the first embodiment, polymer chain 30, for example, may contain the structural unit (second structural unit) represented by formula (34).

Force, such as shear, applied to the macromolecular material of the first embodiment, may dissociate guest groups 20 from host groups R^{H} in the polymer chain, and may cause the macromolecular material to be cut. However, re-adhering the cross-sections of the cut macromolecular material allows guest groups 20 and host groups R^{H} to bind to each other again, thereby forming clathrate complexes. This enables the cross-sections to bind to each other, and repair the macromolecular material. Specifically, when a macromolecular material is the macromolecular material of the first embodiment, the macromolecular material can exhibit excellent self-healing properties.

Fig. 1(b) is a schematic diagram that illustrates a macromolecular material of the second embodiment. The macromolecular material of the second embodiment has a crosslinked structure; i.e., "a three-dimensional network structure." Thus, the macromolecular material of the second embodiment contains, for example, the third structural unit in addition to the structural unit represented by formula (1) in the polymer chain.

In the macromolecular material of the second embodiment, guest groups 20 are bound to polymer chains 30. More specifically, guest groups 20 in the macromolecular material of the second embodiment are included in host groups 10 to form clathrate complexes, and are bound at one end thereof to polymer chains 30 that form the macromolecular material. Additionally, although not shown in Fig. 1, R¹ that binds to host group 10 is bound to polymer chain 30. In other words, the structural unit represented by formula (1) R^{H}-R¹- is bound to polymer chain 30 in the macromolecular material of the second embodiment. Thus, the macromolecular material of the second embodiment has the same structure as that of the macromolecular material of the first embodiment, except that the macromolecular material of the second embodiment forms a crosslinked structure.

The macromolecular material of the second embodiment has a crosslinked structure formed therein, and contains repeated bond of "polymer chain 30"-"guest group 20"---"R^{H}-R¹"-"polymer chain 30," which form polymer chains that constitute the macromolecular material. In this bond, "guest group 20"---"R^{H}-R¹" indicates that guest group 20 and R^{H} forms a clathrate complex. The macromolecular material of the second embodiment is a polymer that has a three-dimensional network structure, and can be referred to as, in particular, a "side-chain-crosslinked macromolecular material."

In the macromolecular material of the second embodiment, polymer chains 30 contain, for example, the structural unit represented by formula (34) (second structural unit) and the third structural unit.

Force, such as shear, applied to the macromolecular material of the second embodiment may dissociate guest groups 20 from host groups R^{H} in the polymer chains, and may cause the macromolecular material to be cut. However, re-adhering the cross-sections of the cut macromolecular material allows guest groups 20 and host groups R^{H} to bind to each other again, thereby forming clathrate complexes. This enables the cross-sections to bind to each other, and repair the macromolecular material. Specifically, when a macromolecular material is the macromolecular material of the second embodiment, the macromolecular material can exhibit excellent self-healing properties.

The guest group in the macromolecular material of the first embodiment and the macromolecular material of the second embodiment is preferably 1-hydroxyadamantane (adamantanol) or 1-adamantaneamine. Polymer chain 30 in the macromolecular material of the first embodiment and the macromolecular material of the second embodiment preferably has the structure represented by formula (34).

Another mode of the macromolecular material is the following macromolecular material of the third embodiment.

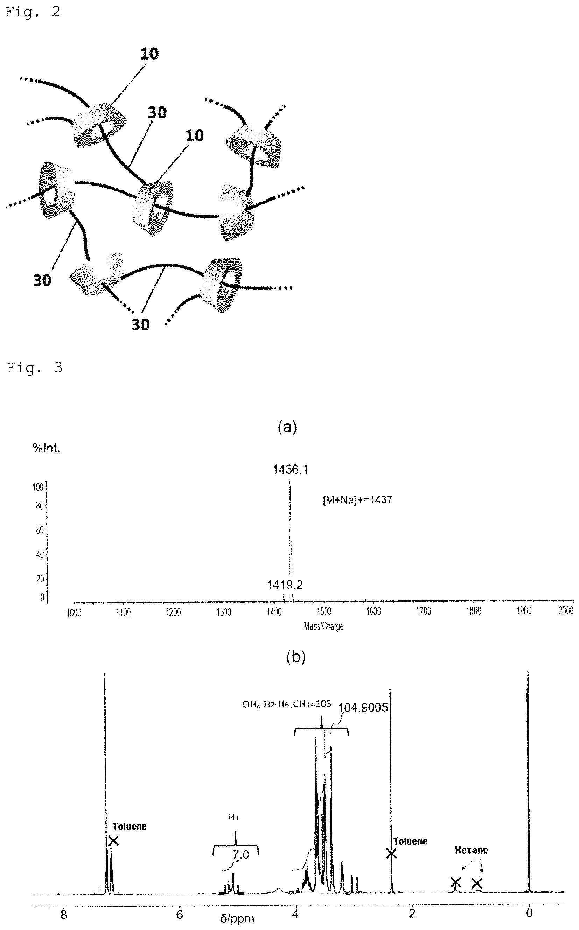

The macromolecular material of the third embodiment has a structure such that the polymer chain penetrates through the ring of the host group. The details are described below with reference to Fig. 2.

Fig. 2 is a schematic diagram that illustrates the macromolecular material of the third embodiment. The macromolecular material of the third embodiment has a crosslinked structure (i.e., a three-dimensional network structure), and also has a structure in which polymer chains 30 penetrate through the rings of host groups 10.

In the macromolecular material of the third embodiment, for example, polymer chain 30 that is included in a host group must be of a size that can penetrate through the ring of the host group. Examples of compounds for forming polymer chain 30 include compounds having two amino groups, compounds having two hydroxy groups, compounds having two carboxy groups, compounds having two epoxy groups, compounds having two isocyanate groups, compounds having one or two thiol groups, and compounds having one or two carboxylic acid chloride. These compounds are all a divalent group, and of a size that can penetrate through the ring of the host group.

The macromolecular material of the third embodiment can also be seen as having one bindable site of a divalent guest group bound to one polymer chain 30, and another bindable site of the divalent guest group to another polymer chain 30. Specifically, divalent guest groups in the macromolecular material of the third embodiment can be seen as intervening between polymer chains to form a single polymer chain 30.

In the macromolecular material of the third embodiment, R¹ that is bound to host group 10 is bound to polymer chain 30, as with the macromolecular materials of the first embodiment and the second embodiment.

As described above, the macromolecular material of the third embodiment has polymer chains 30 forming a crosslinked structure, and has a "polymer chain 30"-"R^{H}-R¹" linkage in this crosslinked structure. Additionally, due to polymer chain 30 penetrating through the ring of host group 10, host group 10 can slide while containing polymer chain 30 therein in the macromolecular material of the third embodiment. Thus, the macromolecular material of the third embodiment can also be referred to as, in particular, a "movable crosslinked macromolecular material."

In the macromolecular material of the third embodiment, the structure of polymer chain 30 can be the structure represented by formula (34); in particular, polymer chain 30 may contain two types or two or more types of R^{A} in the structure represented by formula (34). In this case, polymer chain 30 preferably contains a structural unit derived from 4,4'-dihydroxydiphenylmethane, 4,4'-diaminodiphenylmethane, polyethylene glycol, polypropylene glycol, α,ω-diaminnopolyethylene glycol, or α,ω-diaminopolypropylene glycol. Polymer chain 30 more preferably contains a structural unit derived from 4,4'-dihydroxydiphenylmethane or 4,4'-diaminodiphenylmethane, and particularly preferably contains a structural unit that is 4,4'-dihydroxydiphenylmethane.

In the macromolecular material of the third embodiment, host group 10 can slide while containing polymer chain 30 therein. Thus, the macromolecular material of the third embodiment is excellent in, for example, stress-relaxation action; and exhibits high toughness and strength, thus being excellent in mechanical properties.

Whether a macromolecular material is a movable crosslinked macromolecular material can be determined, for example, from the results of a swelling test of the macromolecular material. For example, a movable crosslinked macromolecular material that has been prepared without using a chemical crosslinking agent, and that has been added to a solvent is determined to be in the form of a movable crosslinked macromolecular material when a swelling phenomenon is observed without the material being dissolved, and determined to be not in the form of a movable crosslinked macromolecular material when the material is dissolved.

The shape of the macromolecular material can be any shape. The macromolecular material can be in various forms, such as membranes, films, sheets, particles, plates, blocks, pellets, powder, foamed materials, or liquid.

The macromolecular material can have a variety of applications. The macromolecular material can be used in a variety of components, such as in automobiles, adhesives, electric components, building components, food containers, and shipping containers.

### 2. A Method for Producing a Macromolecular Material

The method for producing a macromolecular material is not particularly limited.

For example, the macromolecular material may include reacting a clathrate compound with a polyfunctional compound,
the clathrate compound containing
a compound represented by the following formula (2): R²-R^{H} (2) wherein R² represents NH₂ or OH, and R^{H} represents a host group and
a guest molecule.

Below, the step of reacting a clathrate compound with a polyfunctional compound is referred to as a "polymerization step."

The host group is a monovalent group formed by removing one hydrogen atom or hydroxy group from a cyclodextrin derivative, and the cyclodextrin derivative has a structure formed such that a hydrogen atom of at least one hydroxy group contained in a cyclodextrin is replaced with at least one group selected from the group consisting of a hydrocarbon group, an acyl group, and -CONHR wherein R represents a methyl group or an ethyl group.

In formula (2), R^{H} represents a host group, and is synonymous with host group R^{H} in formula (1). All of the preferable embodiments for R^{H} in formula (1) also apply to R^{H} in formula (2). Thus, in formula (2), the acyl group in host group R^{H} is preferably an acetyl group.

Specific examples of compounds represented by formula (2) include a compound represented by the following formula (21).

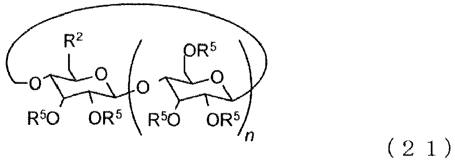

R² in formula (21) is equivalent to R² in formula (2) . Specifically, R² represents NH₂ or OH, R⁵ represents a hydrocarbon group etc., and n represents 5, 6, or 7.

In formula (21), all R⁵s (a hydrocarbon group etc.) may be an identical member selected from the group consisting of a methyl group, an acetyl group, a methyl carbamate group, and an ethyl carbamate group. In formula (21), the hydrocarbon group etc., is preferably a methyl group in every occurrence.

The compound represented by formula (21) includes 6-monodeoxy-6-monoamino-trimethyl-cyclodextrin, 6-monohydroxy-trimethyl-cyclodextrin, 6-monodeoxy-6-monoamino-triacetyl-cyclodextrin, 6-monohydroxy-triacetyl-cyclodextrin, 6-monodeoxy-6-monoamino-triethylcarbamate-cyclodextrin, and 6-monohydroxy-triethylcarbamate-cyclodextrin.

The type of guest molecule of the clathrate compound for use in the polymerization step is the same as that of the guest molecule for forming a guest group described in the production method in the "Macromolecular Material" section above. Thus, the guest molecule for use in the polymerization step can be any guest molecule that can form a clathrate complex with the host group of the compound represented by formula (2); and includes compounds having one or two amino groups, compounds having one or two hydroxy groups, compounds having one or two carboxy groups, compounds having one or two epoxy groups, compounds having one or two isocyanate groups, compounds having one or two thiol groups, and compounds having one or two carboxylic acid chloride.

The method for producing the clathrate compound for use in the polymerization step can also be any method, and can be selected from a wide range of known production methods for clathrate compounds. For example, a clathrate compound can be produced by a method including the step of mixing a compound represented by formula (2) with a guest molecule to obtain a clathrate compound.

In the step of obtaining a clathrate compound, the ratio in mixture of the compound represented by formula (2) to the guest molecule (molar ratio, compound represented by formula (2):guest molecule) is, for example, 10:1 to 1:10, preferably 3:1 to 1:3, and more preferably 2:1 to 1:2. The ratio of the compound represented by formula (2) to the guest molecule may also be 1:1 (molar ratio).

The polyfunctional compound for use in the polymerization step is, for example, the guest molecule described above. Specifically, the polyfunctional compound for use in the polymerization step can be the same compound as the guest molecule capable of forming the clathrate compound. The polyfunctional compound for use may be the same type of guest molecule that forms the clathrate compound, or a different type. The polyfunctional compound for use in the polymerization step may also be a compound other than the guest molecule.

Such a polyfunctional compound that is other than the guest molecule is, for example, at least one compound selected from the group consisting of compounds having two or more amino groups, compounds having two or more hydroxy groups, compounds having two or more carboxy groups, compounds having two or more epoxy groups, compounds having two or more isocyanate groups, carbonyl chloride, and diphenyl carbonate.

Examples of compounds having two or more amino groups include 4,4'-diaminodiphenylmethane, 3,3'-diaminodiphenyl ether, 4,4'-diaminodiphenyl ether, 3,4'-diaminodiphenyl ether, 3,3'-diaminodiphenyl methane, 3,4'-diaminodiphenyl methane, o-phenylenediamine, m-phenylenediamine, p-phenylenediamine, 1,2-diaminoethane, 1,3-diaminopropane, 1,4-diaminobutane, 1,5-diaminopentane, 1,6-diaminohexane, 1,7-diaminoheptane, and 1,8-diaminooctane. These compounds can be used singly, or in a combination of two or more.

Examples of compounds having two or more hydroxy groups include 4,4'-dihydroxydiphenylmethane, 1,3-propanediol, 1,3-butanediol, 2,3-butanediol, 1,4-butanediol, ethylene glycol, diethylene glycol, triethylene glycol, tetraethylene glycol, propylene glycol, dipropylene glycol, trimethylene glycol, and bisphenol A. In particular, when the polyfunctional compound is a compound having three or more hydroxy groups (or just three hydroxy groups), the type of the compound having three or more hydroxy groups may be the same as that of the compound having three or more hydroxy groups described above. These compounds can be used singly, or in a combination of two or more.

Examples of compounds having two or more carboxy groups include adipic acid, malonic acid, succinic acid, and glutaric acid. These compounds can be used singly, or in a combination of two or more.

Examples of compounds having two or more epoxy groups include polyalkylene glycol diglycidyl ether, such as polyethylene glycol diglycidyl ether, and poly-1-methylethylene glycol diglycidyl ether. The polyalkylene glycol diglycidyl ether may have a number average molecular weight (Mn) of 100 to 100000. These compounds can be used singly, or in a combination of two or more.

Examples of compounds having two or more isocyanate groups include 4,4'-diphenylmethane diisocyanate (MDI), 1,6-hexamethylene diisocyanate (HDI), and 2,4-tolylene diisocyanate (TDI). In particular, when the polyfunctional compound is a compound having three or more isocyanate groups (or just three isocyanate groups), the type of the compound having three or more isocyanate groups may be the same as that of the compound having three or more isocyanate groups described above. These compounds can be used singly, or in a combination of two or more.

When the polyfunctional compound contains a compound having three or more hydroxy groups (or just three hydroxy groups) and a compound having three or more isocyanate groups (or just three isocyanate groups), the obtained macromolecular material can form a crosslinked structure in which a three-dimensional network structure is present. For example, such a polyfunctional compound can provide the macromolecular material of the second embodiment or the macromolecular material of the third embodiment, and is particularly preferable to obtain the macromolecular material of the second embodiment. The type of the compound having three or more hydroxy groups is the same as that of the compound having three or more hydroxy groups described above. The type of the compound having three or more isocyanate groups is the same as that of the compound having three or more isocyanate groups described above.

The polyfunctional compound for use may be one type of polyfunctional compound, or may be a combination of two or more types of polyfunctional compound. For example, the polyfunctional compound may be formed only of bifunctional compounds or trifunctional compounds, or a combination of bifunctional compounds and trifunctional compounds. When the polyfunctional compound is composed only of bifunctional compounds, the polyfunctional compound may be formed of different two types of bifunctional compounds. In this case, the ratio of these two functional compounds for use may be, for example, 1:2 to 2:1. Specifically, the polyfunctional compound may be a combination of one type of compound having two isocyanate groups and one type of compound having two hydroxy groups. When the polyfunctional compound for use is a combination of bifunctional compounds and trifunctional compounds, the amount of the trifunctional compounds for use may be 20 mol% or less, preferably 10 mol% or less, and more preferably 5 mol% or less, based on the total amount of the polyfunctional compound. The polyfunctional compound for use may also be a combination of one type of compound having two isocyanate groups, one type of compound having two hydroxy groups, and one type of compound having three hydroxy groups.

In the production method according to the present invention, the polyfunctional compound is preferably a compound different from the guest molecule that forms the clathrate compound.

In the polymerization step, the conditions under which the clathrate compound is reacted with the polyfunctional compound are not particularly limited; and can be selected, for example, from a wide range of known polymerization reactions. For example, a wide range of known polymerization methods such as a known epoxy resin polymerization method or urethane resin polymerization method can be used.

The proportion of the clathrate compound and the polyfunctional compound for use may be as follows: for example, the clathrate compound is used in an amount of 0.1 to 20 mol%, and the polyfunctional compound is used in an amount of 80 to 99.9 mol%. Preferably, the clathrate compound is used in an amount of 0.1 to 5 mol%, and the polyfunctional compound is used in an amount of 95 to 99.9 mol%.

In reacting the clathrate compound with the polyfunctional compound in the polymerization step, a polymerization initiator may be used. The type of the polymerization initiator for use is not limited, and can be any known polymerization initiator. The polymerization initiator (polymerization catalyst) for use can be selected from a wide range of known polymerization initiators for use in, for example, condensation polymerization; and examples include 2-dimethylamino ethyl ether, 4,4'-(3-oxapentan-1,5-diyl)bismorpholine, (1.4-diazabicyclo[2.2.2]octane, bis[(2-dimethylamino)ethyl]ether, (1,1,6,6-tetramethyl hexanediamine), 2-(dimethylamino)ethanol, 1,5-diazabicyclo[4.3.0]-5-nonene, 2,6,10-trimethyl-2,6,10-triazaundecane, N,N,N'-trimethyl-N'-(2-hydroxyethyl)bis(2-amino ethyl) ether, N,N,N',N",N"-pentamethyldiethylenetriamine, N,N,N',N'-tetramethyl-1,6-diaminohexane, N,N,N',N'-tetramethyl-1,4-diaminobutane, 3,3'-iminobis(N,N-dimethylpropylamine), 2-[2-(dimethylamino)ethoxy]ethanol, N,N-dimethylbenzylamine, triethylene diamine, N-methyl morpholine, N,N-dimethyldidodecylamine, N-dodecyl morpholine, N,N-dimethyl cyclohexylamine, N-ethyl morpholine, and dimethylethanolamine. The polymerization initiator (polymerization catalyst) for use may also be an organic metal, such as dibutyltin diacetate, dibutyldimethoxytin, tin 2-ethylhexanoate, dibutyltin dilaurate, or tetrakis(2,4-pentanedionato)zirconium(IV). The amount of the polymerization catalyst for use may be, for example, 0.5 to 5 wt%, based on the total amount of the clathrate compound and the polyfunctional compound.

In reacting the clathrate compound with the polyfunctional compound in the polymerization step, a solvent may be used. The type of the solvent for use can be any type. The amount of the solvent for use can also be any amount. Alternatively, the reaction in the polymerization step may be performed in the absence of a solvent.

The reaction temperature in the polymerization step may be 0 to 500°C. For example, when the obtained macromolecular material is a polyurethane resin, the reaction temperature is preferably 40 to 70°C.

The reaction time in the polymerization step can be any time; and may be 1 minute to 24 hours, and preferably 1 hour to 24 hours.

In the polymerization step described above, a target macromolecular material is obtained. After the polymerization step, treatment such as purification may optionally be performed.

In the production method including the polymerization step, the use of at least one member selected from the group consisting of compounds having one amino group, compounds having one hydroxy group, compounds having one carboxy group, compounds having one epoxy group, compounds having one isocyanate group, compounds having one thiol group, and compounds having one carboxylic acid chloride as a guest molecule provides either the macromolecular material of the first embodiment or the macromolecular material of the second embodiment. In this case, when a compound having three hydroxy groups is not used as a polyfunctional compound, the macromolecular material of the first embodiment can be obtained. When a compound having three hydroxy groups is used as a polyfunctional compound, the macromolecular material of the second embodiment is obtained.

In the production method including the polymerization step, as a guest molecule, the use of at least one compound of a size that can penetrate through the ring of the host group and that is selected from the group consisting of compounds having two amino groups, compounds having two hydroxy groups, compounds having two carboxy groups, compounds having two epoxy groups, compounds having two isocyanate groups, compounds having two thiol groups, and compounds having two carboxylic acid chloride provides the macromolecular material of the third embodiment (i.e., the movable crosslinked macromolecular material described above).

The guest molecule is preferably at least one member selected from the group consisting of compounds having one or two amino groups, and compounds having one or two hydroxy groups. In this case, the reaction in the polymerization step efficiency proceeds, and the obtained macromolecular material is likely to exhibit improved self-healing properties or improved mechanical properties.

The following describes a method for producing the compound represented by formula (2) for use in the polymerization step. The production method is not particularly limited, and can be selected from a wide range of known production methods.

For example, when the target compound represented by formula (2) is a compound wherein R² is NH₂ in formula (2), such a compound represented by formula (2) can be obtained by a production method including the step of adding triphenylphosphine and ammonia water to a cyclodextrin derivative having azide (N₃). The reaction conditions can be any conditions, and may be the same conditions as those for known amination reactions. In this step, various solvents can optionally be used. Examples of solvents include tetrahydrofuran. After a target compound is obtained, the compound can be purified by a suitable method.

The cyclodextrin derivative having azide (N₃) can be produced by a known method. For example, a cyclodextrin derivative having azide (N₃) can be obtained by adding sodium hydride and methyl iodide to a cyclodextrin having azide (N₃) (e.g., 6-deoxy-6-monoazide-β-cyclodextrin). In this reaction, various solvents can optionally be used. Examples of solvents include N,N-dimethylformamide. After the reaction, the product may optionally be subjected to quenching, and further purified by a suitable method.

For example, the production method for a compound represented by formula (2) wherein R² is OH can be any method. Such a compound can be obtained, for example, by hydrolyzing a host-group-containing vinyl monomer described later as a starting material. Hydrolysis can be performed using, for example, strong acid such as trifluoroacetic acid; or, for example, using various organic solvents. Examples of organic solvents include acetone.

Examples of host-group-containing vinyl monomers include the following (h1-1) to (h1-6).

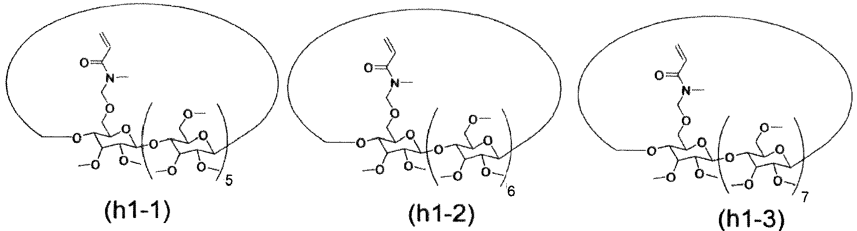

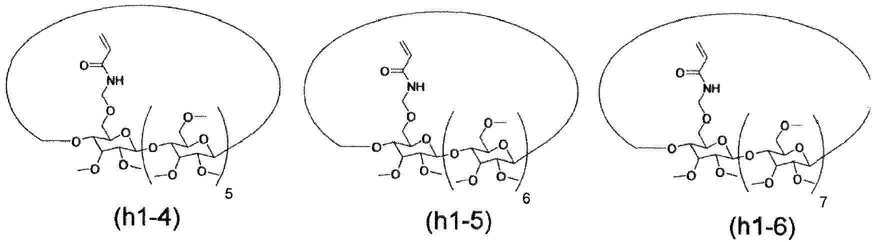

The method for producing a host-group-containing vinyl monomer can be any method. For example, a host-group-containing vinyl monomer can be obtained by substituting vinyl compound A, described later, with a cyclodextrin to prepare a polymerizable cyclodextrin-substitution monomer ("polymerizable CD-substitution monomer" below), and replacing the hydrogen atoms of the hydroxy groups present in the cyclodextrin of this polymerizable CD-substitution monomer with a hydrocarbon group etc.

The method for replacing the hydrogen atoms of the hydroxy groups present in a cyclodextrin with a hydrocarbon group etc., may be selected, for example, from a wide range of known alkylation reactions. For example, replacement of a hydrogen atom with a hydrocarbon group can be performed by reacting halogenated alkyl with the polymerizable CD-substitution monomer in the presence of sodium hydride. In this case, a solution of halogenated alkyl and the polymerizable CD-substitution monomer may be added dropwise to a suspension of sodium hydride. Alternatively, halogenated alkyl, the polymerizable CD-substitution monomer, and sodium hydride may be all mixed together. Examples of halogenated alkyl include methyl iodide, ethyl iodide, and propyl iodide.

The method for replacing the hydrogen atoms of the hydroxy groups present in a cyclodextrin with an acyl group, such as an acetyl group, may be selected, for example, from a wide range of known acylation reactions. For example, replacement of a hydrogen atom with an acetyl group can be performed by reacting halogenated acetyl with the polymerizable CD-substitution monomer in the presence of sodium hydride. In this case, a solution of halogenated acetyl and the polymerizable CD-substitution monomer may be added dropwise to a suspension of sodium hydride. Alternatively, halogenated acetyl, the polymerizable CD-substitution monomer, and sodium hydride may be all mixed together. Examples of halogenated acetyl include acetyl bromide, and acetyl iodide.

Another example of the method for replacing the hydrogen atoms of the hydroxy groups present in a cyclodextrin with an acetyl group is acetylation of the polymerizable CD-substitution monomer using a solvent capable of trapping an acid (e.g., pyridine) in the presence of acetic anhydride or isopropyl acetate.

The method for replacing the hydrogen atoms of the hydroxy groups present in a cyclodextrin with -CONHR wherein R represents a methyl group or an ethyl group may be selected, for example, from a wide range of known alkyl-carbamation reactions. For example, replacement of the hydrogen atoms of the hydroxy groups present in the host group with -CONHR can be performed by reacting the polymerizable CD-substitution monomer in an organic solvent (e.g., DMSO) in the presence of alkyl isocyanate. Examples of alkyl isocyanate include methyl isocyanate, and ethyl isocyanate.

When vinyl compound A is reacted with a cyclodextrin derivative to obtain a host-group-containing vinyl monomer, the cyclodextrin derivative for use may be a compound formed such that the hydrogen atom of at least one hydroxy group of a cyclodextrin is replaced with a hydrocarbon group etc. This cyclodextrin derivative can be obtained, for example, by reacting a cyclodextrin with the halogenated alkyl, halogenated acetyl, or alkyl isocyanate in the presence of sodium hydride.

Vinyl compound A includes the compounds represented by the following formula (5), (6), or (9):

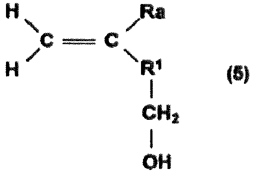

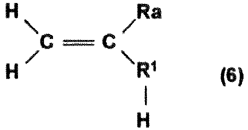

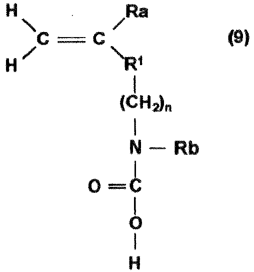

In formulas (5), (6), and (9), Ra represents a hydrogen atom or a methyl group, and R¹ represents a divalent group formed by removing one hydrogen atom from a monovalent group selected from the group consisting of a hydroxy group, a thiol group, an alkoxy group optionally having at least one substituent, a thioalkoxy group optionally having at least one substituent, an alkyl group optionally having at least one substituent, an amino group optionally having one substituent, an amide group optionally having one substituent, an aldehyde group, and a carboxy group. In formula (9), Rb represents hydrogen or a C₁₋₂₀ alkyl group (preferably a C₁₋₁₀ alkyl group, more preferably a C₁₋₆ alkyl group, particularly preferably a methyl group or an ethyl group).

Specific examples of the method for producing the host-group-containing vinyl monomer using vinyl compound A include a method including the step of subjecting vinyl compound A and a cyclodextrin derivative in which the hydrogen atom of at least one hydroxy group is replaced with a hydrocarbon group etc., to dehydration condensation in a solvent, optionally in the presence of an acid catalyst.

Specific examples of the method for producing the host-group-containing vinyl monomer using vinyl compound A also include a method including the step of subjecting vinyl compound A and a cyclodextrin to dehydration condensation in a solvent, optionally in the presence of an acid catalyst. In this method, the target host-group-containing vinyl monomer can be obtained by further performing the step of replacing the hydrogen atom of at least one hydroxy group contained in the product obtained by dehydration condensation with a hydrocarbon group etc. The method for replacing a hydrogen atom with a hydrocarbon group etc., may be the same as the method described above.

Dehydration condensation can be performed, for example, in the presence of an acid catalyst. The acid catalyst can be any catalyst, and can be selected from a wide range of known catalysts. Examples include p-toluenesulfonic acid, aluminum chloride, and hydrochloric acid. The amount of an acid catalyst for use may be, for example, typically 20 mol% or less, and preferably 10 mol% or less based on the cyclodextrin or cyclodextrin derivative; and typically 0.001 mol% or more, preferably 0.01 mol% or more, and more preferably 0.1 mol% or more based on the cyclodextrin or cyclodextrin derivative.

The solvent for use in the reaction can also be any solvent. Examples include water, dimethylformamide, dimethyl sulfoxide, and N-methylpyrrolidone. In particular, from the standpoint of ease of adjusting the concentration of the acid and convenience in controlling the reaction, the solvent is preferably dimethylformamide, dimethyl sulfoxide, or N-methylpyrrolidone, and particularly preferably dimethylformamide. The reaction temperature and reaction time for dehydration condensation are also not limited, and dehydration condensation can be performed under appropriate conditions. From the standpoint of promptly performing the reaction, the reaction temperature is preferably 25 to 90°C, and the reaction time is preferably 1 minute to 3 hours. The reaction time is more preferably 5 minutes to 1 hour. After the reaction, purification may be performed by a known purification technique.

Another method for producing a compound represented by formula (2) wherein R² is OH is the base treatment of Tos-R^{H} (Tos represents a tosyl group, and R^{H} represents a host group). In this case, the base for use may be, for example, MeOH in which Mg is suspended. In MeOH in which Mg is suspended, the amount of Mg may be 1 to 20 wt% relative to MeOh. The conditions for base treatment can be any conditions, and may be the same conditions as those for known base treatments. Another method of base treatment for use may be an operation of refluxing a 1 to 20 wt% NaOH or KOH aqueous solution. Tos-R^{H} for use can be produced by various methods. For example, Tos-R^{H} can be obtained by a method of substituting a tosylated cyclodextrin with a hydrocarbon group etc., a method of substituting a tosylated cyclodextrin with an acetyl group, or a method of substituting a tosylated cyclodextrin with -CONHR wherein R represents a methyl group or an ethyl group.

The method for producing the compound represented by formula (2) is not limited to the methods described above, and the compound represented by formula (2) can also be produced by other known methods. For example, the method using dehydration condensation described above has an advantage in that a host-group-containing vinyl monomer can be produced by a one-step reaction.

### Examples

Below, the present invention is described in more detail with reference to Examples. However, the present invention is not limited to the embodiments of the Examples.

### Production Example 1

In accordance with the synthesis scheme of the following formula (11-1), 6-monohydroxy-trimethyl cyclodextrin ("OHβCDOMe" below) was synthesized.

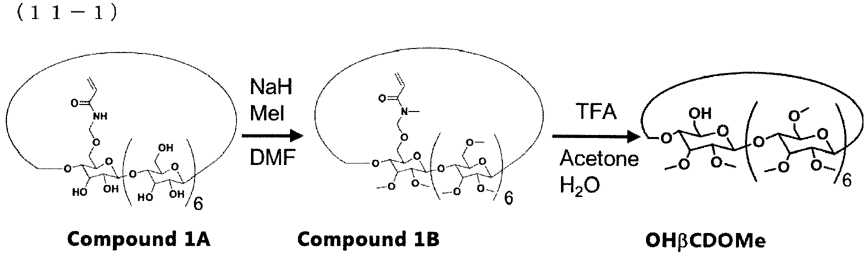

Compound 1A in formula (11-1) was produced as follows. 5 g (4.4 mmol) of β-cyclodextrin, 577 mg (5.7 mmol) of N-hydroxymethylacrylamide, and 73 mg (0.4 mmol) of p-toluenesulfonic acid monohydrate were weighed and placed into a 200-mL round-bottom glass flask. This mixture was then added to 25 mL of N,N-dimethylformamide, thereby preparing a reaction mixture. The reaction mixture was heated to 90°C in an oil bath; and heated with stirring over 1 hour, thereby obtaining a reaction mixture. Subsequently, the reaction mixture was cooled, and poured into 45 mL of strongly stirred acetone. The formed precipitates were filtered, and washed with 10 mL of acetone three times; followed by drying under reduced pressure at room temperature for one hour, thereby obtaining a reaction product. The reaction product was dissolved in 100 mL of distilled water, and passed through a column packed with a porous polystyrene resin (Mitsubishi Chemical Corporation, Diaion HP-20) (apparent density: 600 g/L) to allow for adsorption for 30 minutes. Instead of the column, preparative high-pressure liquid chromatography may be used for separation and purification. Thereafter, the solution component was removed, and 50 mL of a 10% methanol (or acetonitrile) aqueous solution was newly passed through the column three times to wash the polystyrene resin, thereby removing unreacted β-cyclodextrin. Subsequently, 500 mL of a 25% methanol aqueous solution was passed through the column twice to elute acrylamide methyl β-cyclodextrin (compound 1A), which is the desired product. The solvent was removed under reduced pressure, thereby obtaining 810 mg of compound 1A. The yield was about 17%.

Subsequently, 1.0 mmol of compound 1A was weighed in a Schlenk flask, and subjected to nitrogen substitution. To the Schlenk flask, 30 mL of dehydrated N,N-dimethylformamide was added, and the mixture was stirred with ice-cooling; followed by adding 24 mmol of sodium hydride and 26 mmol of methyl iodide, and stirring for 12 hours. Thereafter, 10 mL of methanol and 10 mL of water were added to the Schlenk flask, and quenched. The obtained solution was dried under reduced pressure with an evaporator, and dissolved in 50 mL of a saturated sodium chloride aqueous solution (containing 200 mg of sodium thiosulfate pentahydrate); followed by extraction with 50 mL of toluene three times. The extracted toluene layer was dried and solidified with an evaporator. The obtained solids were dissolved in 50 mL of methanol, and washed with 50 mL of hexane; followed by drying and solidifying the methanol layer with an evaporator, thereby obtaining compound 1B. The obtained compound 1B was dissolved in 20 mL of water and 10 mL of acetone; and then 2.0 mmol of trifluoroacetic acid (TFA) was added thereto, followed by stirring for 1 hour. The obtained solution was dried under reduced pressure with an evaporator, and dissolved in 50 mL of a saturated sodium chloride aqueous solution; followed by extraction with 50 mL of toluene three times. The extracted toluene layer was dried and solidified with an evaporator, thereby obtaining OHβCDOMe.

Fig. 3(a) and Fig. 3(b) respectively illustrate a mass spectrum and an NMR spectrum of OHβCDOMe. From the results of these spectra, the target OHβCDOMe was confirmed to have formed. In OHβCDOMe, all of the hydroxy groups (100%) that were present per molecule of compound 1A were confirmed to have been replaced with a methyl group.

### Production Example 2

First, in accordance with the scheme of the following formula (21-1), NH₂βCDOMe was produced.

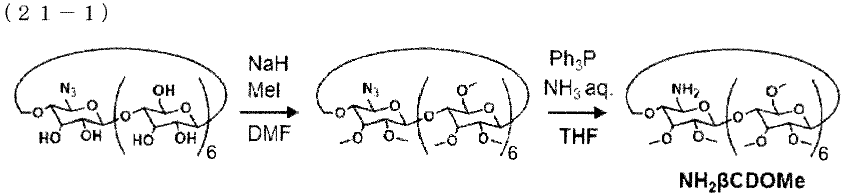

As a starting material of the host molecule, 6.9 mmol of 6-deoxy-6-monoazide-β-cyclodextrin was placed in an eggplant flask; and 500 mL of dehydrated N,N-dimethylformamide was added thereto, followed by stirring with ice-cooling. Further, 150 mmol of sodium hydride and 150 mmol of methyl iodide were added thereto, followed by stirring for 12 hours. Thereafter, 20 mL of methanol was then added to the eggplant flask, and 20 mL of water was further added for quenching. The obtained solution was dried under reduced pressure with an evaporator; and 100 mL of dichloromethane was added thereto, followed by washing it with 50 mL of a saturated sodium hydrogen carbonate aqueous solution, 50 mL of an aqueous solution containing 2.5 g of sodium thiosulfate pentahydrate, and 50 mL of a saturated sodium chloride solution.

To the washed dichloromethane layer, 10 g of magnesium sulfate was added; and the mixture was stirred at room temperature, and filtered. The filtrate was then dried and solidified with an evaporator. The obtained solids were dissolved in 100 mL of tetrahydrofuran; and then 15 mmol of triphenylphosphine and 30 mL of 28% ammonia water were added thereto, followed by stirring at room temperature for 12 hours. After drying under reduced pressure with an evaporator, the obtained dry product was dissolved in 100 mL of dichloromethane, and washed with 100 mL of water and 100 mL of a saturated sodium chloride solution.

To the washed dichloromethane layer, 10 g of magnesium sulfate was added, and the mixture was stirred at room temperature and filtered. The filtrate was then dried and solidified with an evaporator. The obtained solids were purified by silica column chromatography (eluent composition, dichloromethane:methanol = 9:1). This host-group-containing polymerizable monomer is expressed as NH₂βCDOMe.

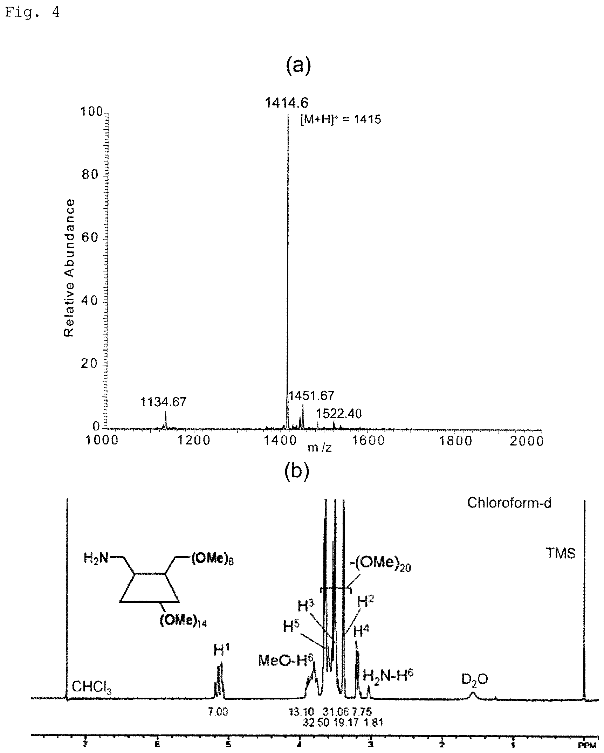

Fig. 4(a) and Fig. 4(b) respectively illustrate a mass spectrum and an NMR spectrum of NH₂βCDOMe. From these results, the target NH₂βCDOMe was confirmed to have formed. In NH₂βCDOMe, all of the hydroxy groups (100%) that were present per molecule of the starting material of the host group were confirmed to have been replaced with a methyl group.

### Tensile Test

Macromolecular materials (thickness: 1 mm) formed from polymers obtained in the following Examples and Comparative Examples were subjected to a tensile-load curve test (Autograph AGX-plus, produced by Shimadzu Corporation). From the obtained tensile-load curve (stress-strain curve), the rupture point of each macromolecular material was observed. With this rupture point taken as the final point, the maximum stress applied until the final point was determined to be the rupture stress of the macromolecular material. This tensile test was performed with the bottom end of the macromolecular material fixed, and the upper end pulled at a tension rate of 0.1 to 1 mm/min (upward operation).

In the tensile-load curve test, when a material exhibits a high value in rupture stress or rupture strain (which may be simply referred to as "strain"), or both, the material can be determined to be excellent in toughness and strength. In particular, when a material exhibits a high value in both rupture stress and rupture strain (or simply "strain"), the material can be determined to be excellent in fracture energy.

### Example 1

OHβCDOMe (the compound represented by formula (2)) obtained in Production Example 1 and adamantanol (guest molecule) were mixed in water at 90°C in a molar ratio of 1:1, and dissolved. The obtained solution was cooled to 25°C, and filtered. The obtained filtrate was then freeze-dried, thereby obtaining a clathrate compound OHβCDOMe-AdOH.

In accordance with the scheme of the following formula (10-1), a macromolecular material was produced using the obtained clathrate compound OHβCDOMe-AdOH.

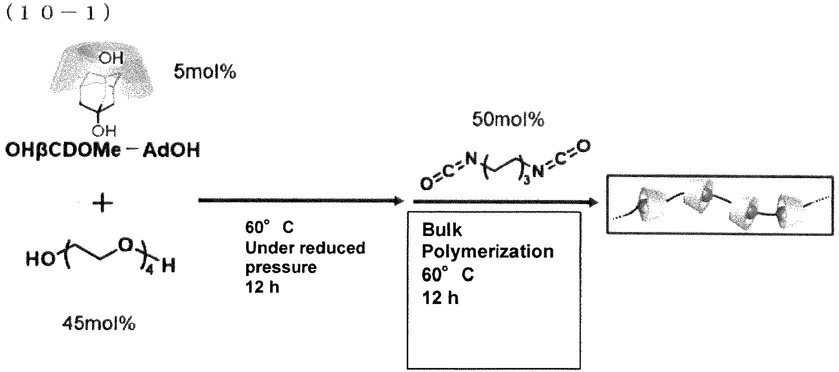

5 mol% of the clathrate compound OHβCDOMe-AdOH and 45 mol% of tetraethylene glycol (polyfunctional compound) were mixed and dried under reduced pressure at 60°C for 12 hours; followed by adding 50 mol% of hexamethylene diisocyanate, thereby obtaining a mixture. This mixture was reacted at 60°C in a nitrogen atmosphere for 12 hours (polymerization step), thereby obtaining a macromolecular material.

### Comparative Example 1

The procedure of Example 1 was repeated, except that OHβCDOMe-AdOH was changed to ethylene glycol, thereby obtaining a macromolecular material.

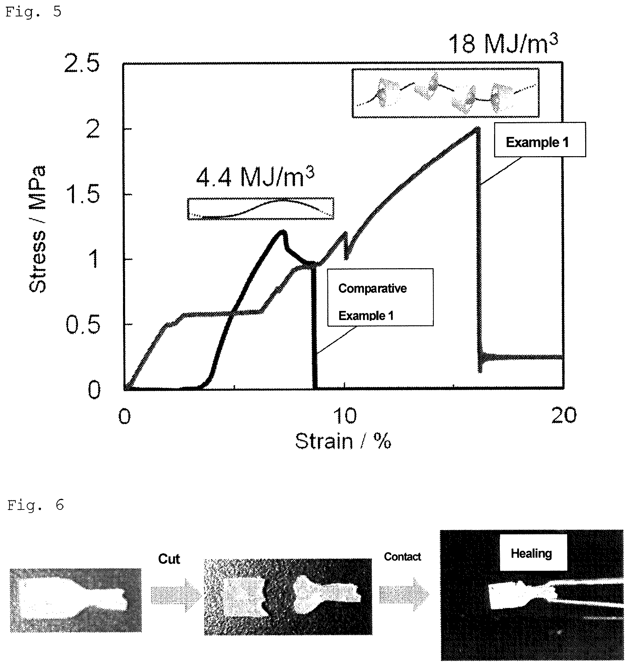

Fig. 5 illustrates the tensile-load curves (stress-strain curves) of the macromolecular materials of Example 1 and Comparative Example 1. The macromolecular material of Example 1 obtained by using the clathrate compound OHβCDOMe-AdOH exhibited toughness superior to that of the macromolecular material of Comparative Example 1 obtained without using a clathrate compound. As seen in Fig. 5, the macromolecular material of Example 1 had a fracture energy of 18 kJ/m³, and the macromolecular material of Comparative Example 1 had a fracture energy of 4.4 kJ/m³. The fracture energy of a macromolecular material as used here refers to the value calculated from the area of the stress-strain curve.

### Evaluation of Self-Healing Properties

Fig. 6 illustrates the results of a self-healing properties test performed on the macromolecular material obtained in Example 1. In this self-healing properties test, the test specimen obtained in Example 1 was cut in the middle, and separated in half. The two pieces of the test specimen were then allowed to be in contact with each other at room temperature (25°C) for 24 hours. Whereas the two cut pieces did not adhere to each other in Comparative Example 1 (not shown), the cut pieces of Example 1 adhered to each other. The adhesive force was enough to support their own weight when the adhered pieces were lifted with tweezers. Thus, the macromolecular material obtained in Example 1 was confirmed to have self-healing properties.

The results above suggest that the macromolecular material obtained in Example 1 was a terminal-crosslinked macromolecular material (the macromolecular material of the first embodiment), as schematically illustrated in formula (10-1).

### Example 2

In accordance with the scheme of the following formula (10-2), a macromolecular material was produced using the OHβCDOMe-AdOH used in Example 1.

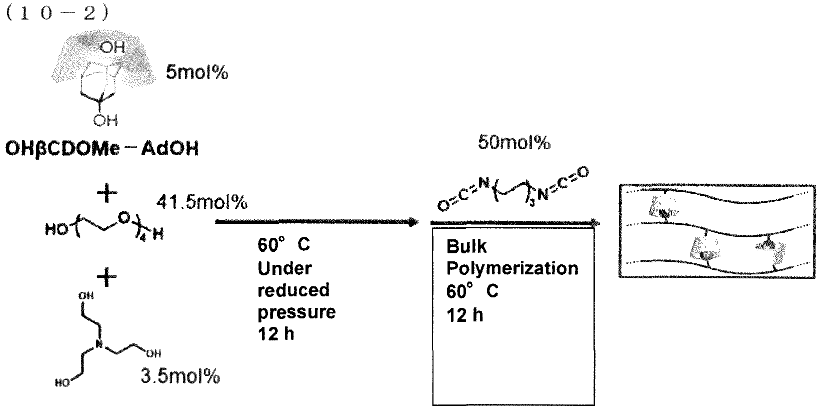

5 mol% of the clathrate compound OHβCDOMe-AdOH, 41.5 mol% of tetraethylene glycol (polyfunctional compound), and 3.5 mol% of triethanolamine (polyfunctional compound) were mixed and dried under reduced pressure at 60°C for 12 hours; followed by adding 50 mol% of hexamethylene diisocyanate, thereby obtaining a mixture. This mixture was reacted at 60°C in a nitrogen atmosphere for 12 hours (polymerization step), thereby obtaining a macromolecular material.

### Comparative Example 2

The procedure of Example 2 was repeated, except that OHβCDOMe-AdOH was changed to ethylene glycol, thereby obtaining a macromolecular material.

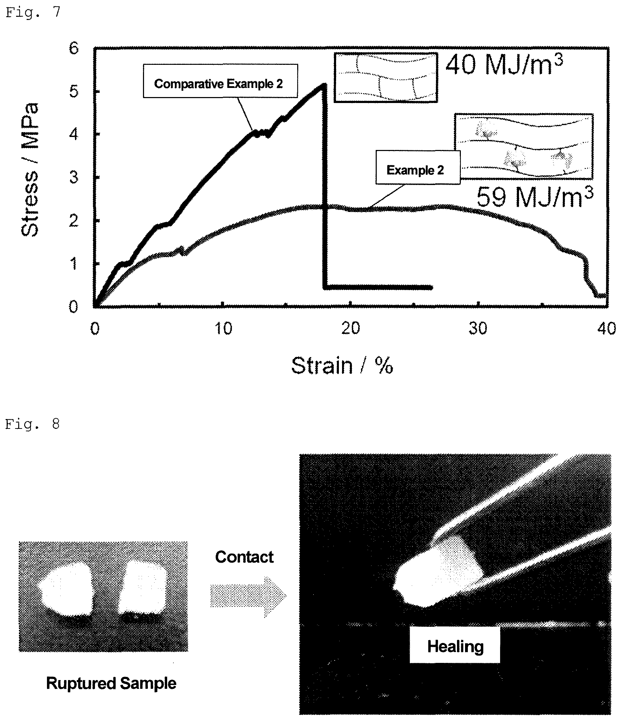

Fig. 7 illustrates the tensile-load curves (stress-strain curves) of the macromolecular materials of Example 2 and Comparative Example 2. The macromolecular material of Example 2 obtained by using the clathrate compound OHβCDOMe-AdOH exhibited toughness superior to that of the macromolecular material of Comparative Example 2 obtained without using a clathrate compound. As seen in Fig. 7, the macromolecular material of Example 2 had a fracture energy of 59 kJ/m³, and the macromolecular material of Comparative Example 2 had a fracture energy of 40 kJ/m³. The fracture energy of a macromolecular material as used here refers to the value calculated from the area of the stress-strain curve.

### Evaluation of Self-Healing Properties

Fig. 8 illustrates the results of a self-healing properties test performed on the macromolecular material obtained in Example 2. In this self-healing properties test, the test specimen obtained in Example 2 was cut in the middle, and separated in half. The two pieces of the test specimen were then allowed to be in contact with each other at room temperature (25°C) for 24 hours. Whereas the two cut pieces did not adhere to each other in Comparative Example 2 (not shown), the cut pieces of Example 2 adhered to each other. The adhesive force was enough to support their own weight when the adhered pieces were lifted with tweezers. Thus, the macromolecular material obtained in Example 2 was confirmed to have self-healing properties.

The results above suggest that the macromolecular material obtained in Example 2 was a side-chain-crosslinked macromolecular material (the macromolecular material of the second embodiment), as schematically illustrated in formula (10-2).

### Example 3

OHβCDOMe (the compound represented by formula (2)) obtained in Production Example 1 and 4,4'-dihydroxydiphenylmethane (a guest molecule of a size capable of penetrating the ring of the host) were mixed in water at 90°C in a molar ratio of 1:1, and dissolved. The obtained solution was cooled to 25°C, and filtered. The obtained filtrate was then freeze-dried, thereby obtaining a clathrate compound OHβCDOMe-BPhOH.

In accordance with the scheme of the following formula (10-3), a macromolecular material was produced using the obtained clathrate compound OHβCDOMe-BPhOH.

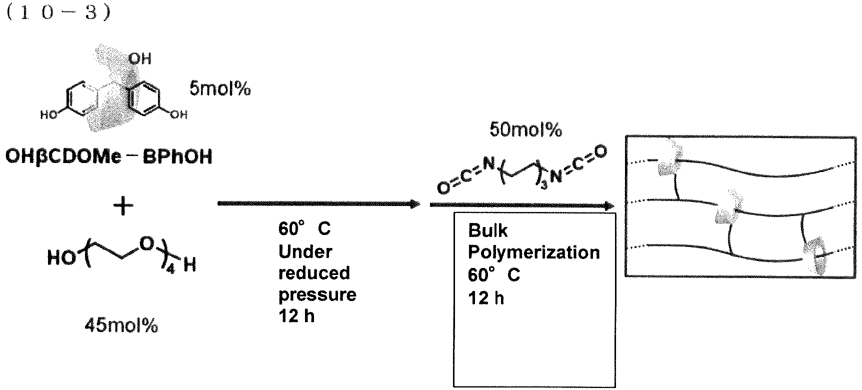

5 mol% of the clathrate compound OHβCDOMe-BPhOH and 45 mol% of tetraethylene glycol (polyfunctional compound) were mixed and dried under reduced pressure at 60°C for 12 hours; followed by adding 50 mol% of hexamethylene diisocyanate, thereby obtaining a mixture. This mixture was reacted at 60°C in a nitrogen atmosphere for 12 hours (polymerization step), thereby obtaining a macromolecular material.

### Comparative Example 3

The procedure of Example 3 was repeated, except that OHβCDOMe-BPhOH was changed to triethanolamine, thereby obtaining a macromolecular material.

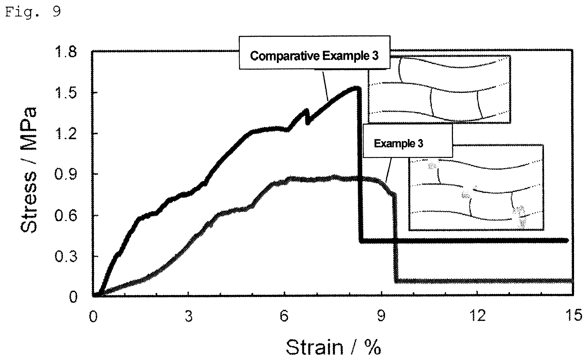

Fig. 9 illustrates the tensile-load curves of the macromolecular materials of Example 3 and Comparative Example 3. The macromolecular material of Example 3 had a strain of 9.8%, and the macromolecular material of Comparative Example 3 had a strain of 8.1%. The macromolecular material of Example 3 obtained by using the clathrate compound OHβCDOMe-BPhOH exhibited toughness superior to that of the macromolecular material of Comparative Example 3 obtained without using a clathrate compound.

The results above suggest that the macromolecular material obtained in Example 3 was a movable crosslinked polymer (the macromolecular material of the third embodiment), as schematically illustrated in formula (10-3).

### Example 4

NH₂βCDOMe (the compound represented by formula (2)) obtained in Production Example 2 and 1-adamantaneamine (guest molecule) were mixed in water at 90°C in a molar ratio of 1:1, and dissolved. The obtained solution was cooled to 25°C, and filtered. The obtained filtrate was then freeze-dried, thereby obtaining a clathrate compound NH₂βCDOMe-AdNH₂.

In accordance with the scheme of the following formula (10-4), a macromolecular material was produced using the obtained clathrate compound NH₂βCDOMe-AdNH₂.

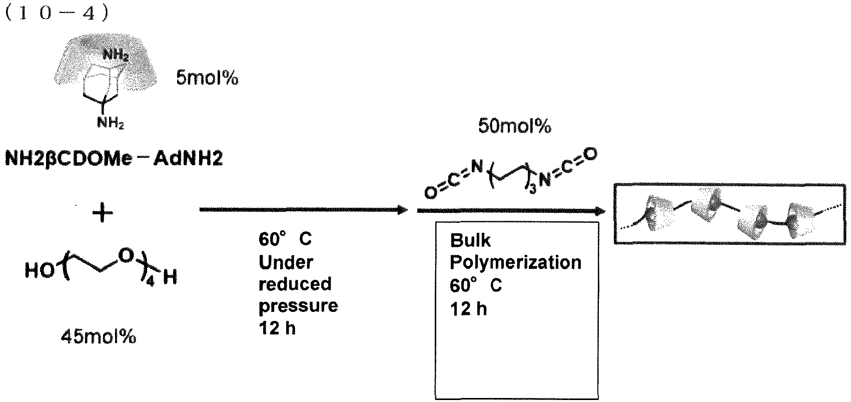

5 mol% of the clathrate compound NH₂βCDOMe-AdNH₂ and 45 mol% of tetraethylene glycol (polyfunctional compound) were mixed and dried under reduced pressure at 60°C for 12 hours; followed by adding 50 mol% of hexamethylene diisocyanate, thereby obtaining a mixture. This mixture was reacted at 60°C in a nitrogen atmosphere for 12 hours (polymerization step), thereby obtaining a macromolecular material.

### Comparative Example 4

The procedure of Example 4 was repeated, except that NH₂βCDOMe-AdNH₂ was not used; and that the amount of tetraethylene glycol was changed to 50 mol%, thereby obtaining a macromolecular material.

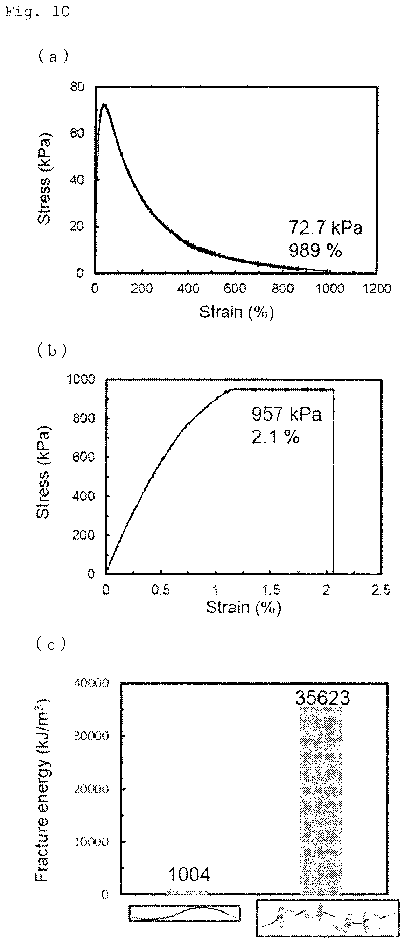

Fig. 10(a) and Fig. 10(b) respectively illustrate the tensile-load curves (stress-strain curves) of the macromolecular materials of Example 4 and Comparative Example 4. Fig. 10(c) illustrates the measurement results of fracture energy of the macromolecular materials of Example 4 (the bar on the right side) and Comparative Example 4 (the bar on the left side). The macromolecular material obtained by using the clathrate compound NH₂βCDOMe-AdNH₂ exhibited toughness superior to that of the macromolecular material of Comparative Example 4 obtained without using a clathrate compound. The macromolecular material of Example 4 had a fracture energy of 35623 kJ/m³, and the macromolecular material of Comparative Example 4 had a fracture energy of 1004 kJ/m³. The fracture energy of a macromolecular material as used here refers to the value calculated from the area of the stress-strain curve.

### Evaluation of Self-Healing Properties

The test specimen obtained in Example 4 was cut in the middle, and separated in half. The two pieces of the test specimen were then allowed to be in contact with each other at room temperature (25°C) for 24 hours. Whereas the two cut pieces of Comparative Example 2 did not adhere to each other (not shown), the cut pieces of Example 4 adhered to each other. The adhesive force was enough to support their own weight when the adhered pieces were lifted with tweezers. Thus, the macromolecular material obtained in Example 4 was confirmed to have self-healing properties.

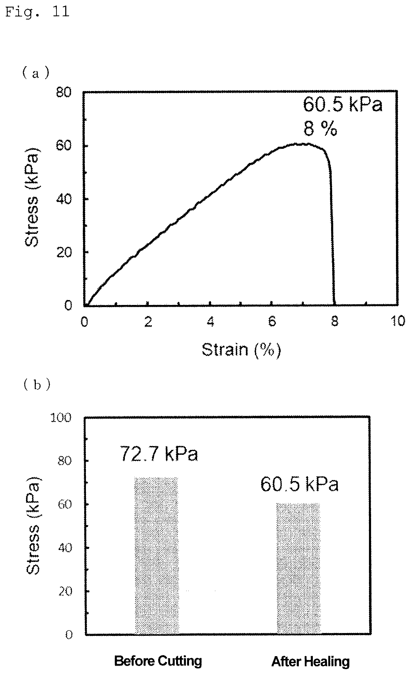

Fig. 11(a) illustrates the tensile-load curve of the repaired test specimen of the macromolecular material obtained in Example 4.

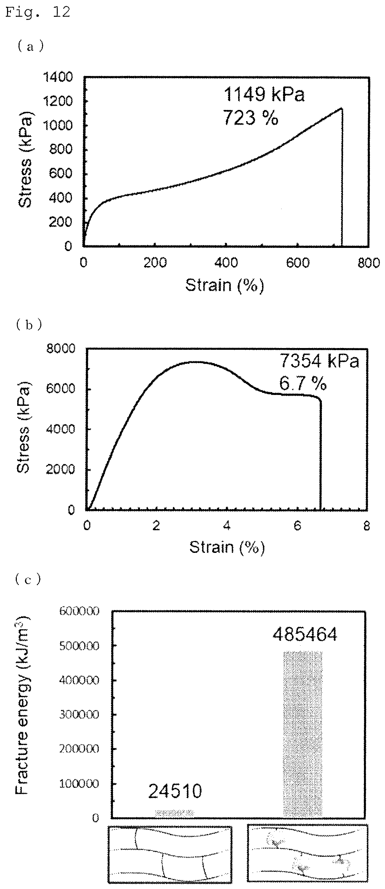

Fig. 11(b) illustrates the results of the rupture stress before cutting and the rupture stress after healing of the macromolecular material obtained in Example 4. The results shown in Fig. 12 indicate that the healed macromolecular material obtained in Example 4 exhibited 83% recovery in rupture stress compared with the macromolecular material before cutting.

The results above suggest that the macromolecular material obtained in Example 4 was a terminal-crosslinked macromolecular material (the macromolecular material of the first embodiment), as schematically illustrated in formula (10-4).

### Example 5

In accordance with the scheme of the following formula (10-5), a macromolecular material was produced using the clathrate compound NH₂βCDOMe-AdNH₂ used in Example 4.

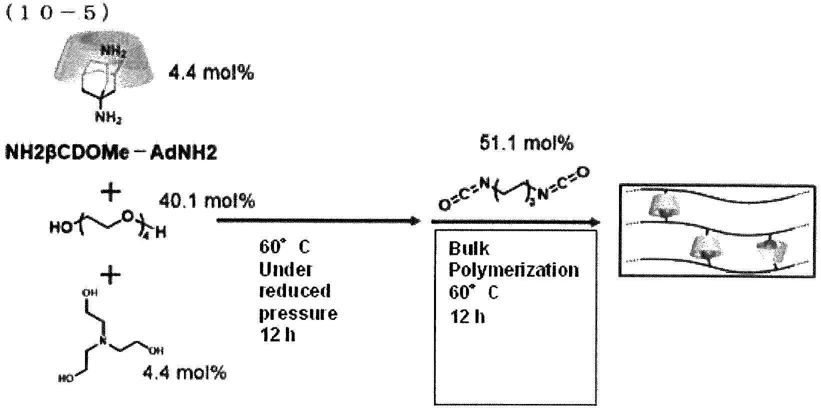

4.4 mol% of the clathrate compound NH₂βCDOMe-AdNH₂, 40.1 mol% of tetraethylene glycol (polyfunctional compound), and 4.4 mol% of triethanolamine (polyfunctional compound) were mixed and dried under reduced pressure at 60°C for 12 hours; followed by adding 51.1 mol% of hexamethylene diisocyanate, thereby obtaining a mixture. This mixture was reacted at 60°C in a nitrogen atmosphere for 12 hours (polymerization step), thereby obtaining a macromolecular material.

### Comparative Example 5

The procedure of Example 5 was repeated, except that NH₂βCDOMe-AdNH₂ was not used; and that the amount of tetraethylene glycol was changed to 44.5 mol%, thereby obtaining a macromolecular material.

Fig. 12(a) and Fig. 12(b) respectively illustrate the tensile-load curves (stress-strain curves) of the macromolecular materials of Example 5 and Comparative Example 5. Fig. 12(c) illustrates the measurement results of fracture energy of the macromolecular materials of Example 5 (the bar on the right side) and Comparative Example 5 (the bar on the left side). The macromolecular material obtained by using the clathrate compound NH₂βCDOMe-AdNH₂ of Example 5 exhibited toughness superior to that of the macromolecular material of Comparative Example 5 obtained without using a clathrate compound. The macromolecular material of Example 5 had a fracture energy of 485464 kJ/m³, and the macromolecular material of Comparative Example 5 had a fracture energy of 24510 kJ/m³. The fracture energy of a macromolecular material as used here refers to the value calculated from the area of the stress-strain curve.

### Evaluation of Self-Healing Properties

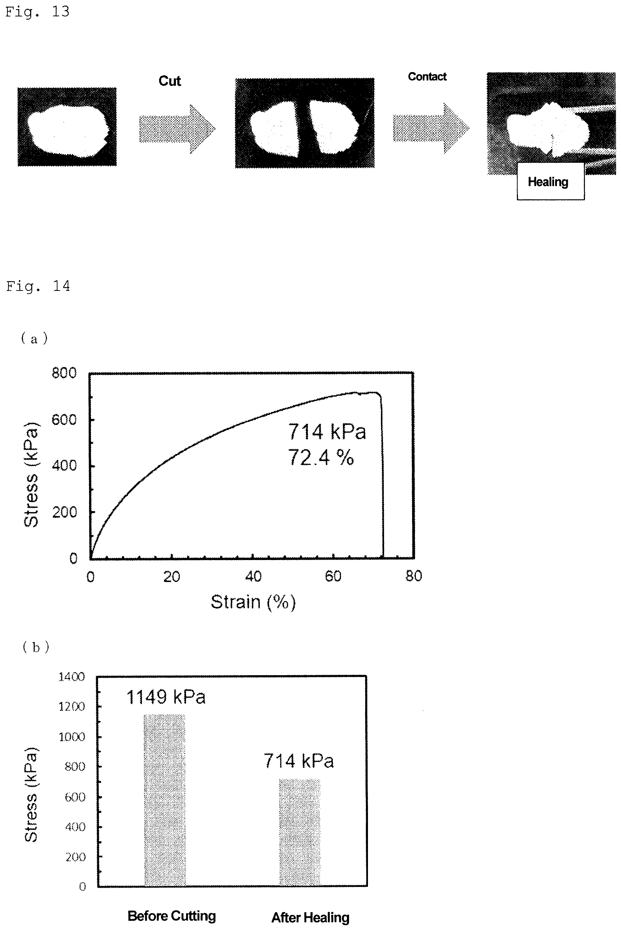

Fig. 13 illustrates the results of the self-healing properties test performed on the macromolecular material obtained in Example 5. In this self-healing properties test, the test specimen obtained in Example 5 was cut in the middle, and separated in half. The two pieces of the test specimen were then allowed to be in contact with each other at room temperature (25°C) for 24 hours. Whereas the two cut pieces of Comparative Example 5 did not adhere to each other (not shown), the cut pieces of Example 5 adhered to each other. The adhesive force was enough to support their own weight when the adhered pieces were lifted with tweezers. Thus, the macromolecular material obtained in Example 5 was confirmed to have self-healing properties.

Fig. 14(a) illustrates the tensile-load curve of the test specimen of the macromolecular material obtained in Example 5.

Fig. 14(b) illustrates the results of the rupture stress before cutting and the rupture stress after healing of the macromolecular material obtained in Example 5. The results shown in Fig. 13 indicate that the healed macromolecular material obtained in Example 5 exhibited 62% recovery in rupture stress compared with the macromolecular material before cutting.

The results above suggest that the macromolecular material obtained in Example 5 was a side-chain-crosslinked macromolecular material (the macromolecular material of the second embodiment), as schematically illustrated in formula (10-5) .

### Description of the Reference Numerals

10: host group
20: guest group
30: polymer chain

## Claims

1. A macromolecular material comprising in a polymer chain thereof a structural unit represented by the following formula (1) :
-R¹-R^{H} (1)
wherein R¹ represents NH or O, and R^{H} represents a host group,
wherein
the host group is a monovalent group formed by removing one hydrogen atom or hydroxy group from a cyclodextrin derivative, and
the cyclodextrin derivative has a structure formed such that a hydrogen atom of at least one hydroxy group contained in a cyclodextrin is replaced with at least one group selected from the group consisting of a hydrocarbon group, an acyl group, and -CONHR wherein R represents a methyl group or an ethyl group.

2. The macromolecular material according to claim 1, wherein the acyl group is an acetyl group.

3. The macromolecular material according to claim 1 or 2, wherein the host group includes a guest group to form a clathrate complex, and the guest group has a structure such that the guest group is chemically bonded to the polymer chain.

4. The macromolecular material according to claim 1 or 2, having a structure such that the polymer chain penetrates through a ring of the host group.

5. A method for producing a macromolecular material comprising reacting a clathrate compound with a polyfunctional compound,
the clathrate compound containing
a compound represented by the following formula (2):
R²-R^{H} (2)
wherein R² represents NH₂ or OH, and R^{H} represents a host group and
a guest molecule,
wherein
the host group is a monovalent group formed by removing one hydrogen atom or hydroxy group from a cyclodextrin derivative, and
the cyclodextrin derivative has a structure formed such that a hydrogen atom of at least one hydroxy group contained in a cyclodextrin is replaced with at least one group selected from the group consisting of a hydrocarbon group, an acyl group, and -CONHR wherein R represents a methyl group or an ethyl group.

6. The method for producing a macromolecular material according to claim 5, wherein the acyl group is an acetyl group.

7. The method for producing a macromolecular material according to claim 5 or 6, wherein the guest molecule is at least one member selected from the group consisting of compounds having one or two amino groups, and compounds having one or two hydroxy groups.
